# EUROPÄISCHE PATENTSCHRIFT

(11) **EP 3 461 251 B1**
(45) Veröffentlichungstag und Bekanntmachung des Hinweises auf die Patenterteilung: **27.10.2021**
(21) Anmeldenummer: 17788846.8
(22) Anmeldetag: 24.04.2017
(51) Int. Cl.: A45C 15/06, A61N 5/06, F21V 33/00

(54) **GEPÄCKSTÜCK MIT CIRCADIAN DYNAMISCH WIRKSAMER LICHTQUELLE**
PIECE OF LUGGAGE WITH A LIGHT SOURCE BEING DYNAMICALLY ACTIVE IN CIRCADIAN MANNER
BAGAGE COMPRENANT UNE SOURCE LUMINEUSE À ACTION DYNAMIQUE CIRCADIENNE

(30) Priorität: 25.04.2016 DE 202016002661 U
(43) Veröffentlichungstag der Anmeldung: 03.04.2019
(73) Patentinhaber: Kunz, Dieter, 14163 Berlin (DE); Hädel, Sven, 16547 Birkenwerder (DE)
(72) Erfinder: Kunz, Dieter, 14163 Berlin (DE); Hädel, Sven, 16547 Birkenwerder (DE)
(74) Vertreter: Kayser, Christoph
(86) Internationale Anmeldenummer: PCT/DE2017/000112
(87) Internationale Veröffentlichungsnummer: WO 2017/186206

(56) Entgegenhaltungen:
- DE-A1-102013 221 723
- DE-U1-202015 004 814
- JP-U- 3 167 823
- US-A1- 2005 270 764
- US-A1- 2009 316 426
- US-A1- 2014 268 784

## Beschreibung

Die vorliegende Erfindung betrifft ein Gepäckstück mit wenigstens einem Gehäuse, in welches eine Beleuchtungseinrichtung integriert ist, mit einem ersten Gehäuseteil und einem zweiten Gehäuseteil, die wenigstens teilweise lösbar miteinander verbunden sind, und mit einer Steuereinrichtung zur Steuerung der Beleuchtungseinrichtung.

### Ein solches Gepäckstück ist aus dem Stand der Technik zum Beispiel aus US 2005/0270764 A1, JP 2 167823 U oder US 2009/0316426 A1 bekannt.

Hintergrund der vorliegenden Erfindung ist folgender Sachverhalt: Im Jahr 2001 wurde in einigen Ganglienzellen der Netzhaut des Auges ein Fotopigment identifiziert, dem man den Namen Melanopsin gegeben hat. Zeitgleich wurde gezeigt, dass Melanopsin auf Licht im Wesentlichen aus dem sichtbaren blauen Bereich reagiert und auf rotes Licht nicht. In kurzer Folge wurde zuerst im Tier-, später auch im Humanexperiment eine Reihe von erstaunlich ausgeprägten Wirkungen gezeigt. Zum einen besteht eine akute Wirkung im Sinne der Aktivierung des Gehirns, was eine akute Leistungssteigerung mit verbesserter Konzentration, gesteigerter Aufmerksamkeit, verbesserter kognitiver Leistung und auch physischer Leistungsbereitschaft hervorruft. Den Effekt kennt jeder, der morgens in einem dunklen Raum das Einschlafen kaum verhindern kann, und sobald er vor die Tür geht, hellwach ist. Zum anderen besteht ein subakuter Effekt über das System an inneren Uhren. Dieser tritt erst nach Stunden auf, teilweise auch erst nach Tagen. Dies macht evolutionsbiologisch Sinn, da über das System an inneren Uhren Saisonalität vermittelt wird. Saisonales Brüten, Winterschlaf und Vogelzug finden ohne das circadiane System nicht statt. Ein einzelner grauer Tag sollte diese Veränderung in der Biologie nicht induzieren können.

Jeder kennt das Phänomen, dass wenn er in die USA fliegt, eine Umstellung innerhalb von einem Tag nicht möglich ist. Es ist gut nachgewiesen, dass bei optimalem Einsatz der Zeitgeber zum richtigen Zeitpunkt der Mensch um maximal 1,5 Stunden pro Tag zu verschieben ist. Das heißt, sich von einer Zeit in Berlin auf eine Zeit in New York umzustellen, ist schneller als innerhalb von vier Tagen nicht möglich.

Die Identifikation von Melanopsin und die Beschreibung seines Aktionsspektrums vor 15 Jahren legten einfache Schlüsse nahe: Aktivierendes Licht muss blau sein. Je mehr Blau, desto besser. Dies gilt für den regulären Arbeitsplatz, für Schulen, Krankenhäuser und öffentliche Gebäude. Und damit bei der Nachtschicht niemand einschläft, sollte auch hier im Wesentlichen blau-angereichertes Licht eingesetzt werden.

Einige Versuche waren vielversprechend, z. B. beim Einsatz von hellem, blauem Licht in Pflegeheimen, andere funktionierten gar nicht. Es stellte sich heraus, dass es Nebeneinflüsse gibt. Der Zeitpunkt der Beleuchtung ist von überragender Bedeutung. "Blaues Licht" am Morgen wirkt sehr anders als abends und nachts. Die Beleuchtung der Vortage hat Einfluss auf die Wirkung von Licht. Auch der Schlaf der Vornacht hat Einfluss auf die Wirkung von Licht. Ob man während der Beleuchtung Kaffee trinkt oder nicht, hat einen Einfluss auf die Wirkung. Menschen mit blauen Augen reagieren anders auf Licht als Menschen mit braunen Augen.

Bei dem eingangs genannten Gepäckstück **aus** US 2005/0270764 A1**,** JP 2 167823 U **oder** US 2009/0316426 A1 **handelt** es sich zum Beispiel um **ein als Tasche oder** Koffer **ausgebildetes Gepäckstück,** in dem eine Leuchte angeordnet ist, um dessen Inhalt zu beleuchten. Eine solche Leuchte, die in einem Gepäckstück integriert ist, könnte aber auch als Leseeinrichtung vorgesehen sein, um dem Benutzer das Lesen eines Schriftstücks oder Buches zu erleichtern. Alle Leuchten oder Beleuchtungseinrichtungen, die in **den** Gepäckstücken zum Einsatz kommen, haben einen Lichtanteil im blauen Spektralbereich von 450 nm bis 490 nm und verfügen somit über einen Weißlichtanteil, der in dem Gehirn eines Benutzers das Signal für einen Wachzustand auslöst. **Selbst bei einer zur Verfügung stehenden ästhetischen Farbwahl, ist üblicherweise ein Weißlichtanteil in der jeweils eingestellten Farbe. Der Weißlichtanteil ist im Stand der Technik nicht kritisch oder problematisch. Dieser** ist für den Benutzer besonders **aber** in den Abend- und Nachtstunden und auch bei Reisen über verschiedene Zeitzonen problematisch. Solche künstlichen Leuchten oder Beleuchtungseinrichtungen unterstützen den natürlichen Schlaf-Wach-Rhythmus eines Benutzers nicht, **jedenfalls nicht durch geplantes Handeln.**

Die aus dem Stand der Technik bekannten Leuchten und Beleuchtungseinrichtungen können nicht dazu verwendet werden, circadiane Rhythmen eines Benutzers zu beeinflussen, da sie nicht je nach Tageszeit, Ortszeit sowie intraindividueller Präferenz und Notwendigkeit unterschiedliche Lichtszenarien in Bezug auf z.B. Beleuchtungsstärke, Beleuchtungsfarbe und spektraler Verteilung emittieren können.

Die Aufgabe der vorliegenden Erfindung ist daher, ein Gepäckstück mit einer Beleuchtungseinrichtung derart weiter zu entwickeln, dass diese die über das Auge vermittelte, nichtvisuell wirksamen Lichtwirkungen eines Benutzers beeinflussen kann.

Die Aufgabe der vorliegenden Erfindung wird dadurch gelöst, dass **die Steuereinrichtung die Beleuchtungseinrichtung derart steuert, dass diese den circadianen Rhythmus des Benutzer verschiebt, wobei die Beleuchtungseinrichtung eine mehrkanalige Lichtquelle mit** wenigstens einen Melanopsin aktivierenden Lichtkanal und wenigstens einen Melanopsin nicht aktivierenden Kanal umfasst.

Mit der erfindungsgemäßen Beleuchtungseinrichtung in einem Gepäckstück kann der Benutzer des Gepäckstücks Melanopsin vermittelte Wirkungen zeitzonenunabhängig unterstützen. Wenn zum Beispiel ein Sportler nach Südamerika zu einem Wettkampf reisen muss, kann dieser bereits ein paar Tage vor der Abreise das Gepäckstück in seinem Zimmer benutzen und mithilfe der Steuereinrichtung auf die Zeitzone in Südamerika einstellen, um seinen circadianen Rhythmus zu verschieben. Bei seiner Ankunft in Südamerika kann der Sportler das Gepäckstück über die Steuereinrichtung so einstellen, dass sein Hotelzimmer mit optimalem Licht beleuchtet wird. Dadurch kann der Sportler die negativen körperlichen Auswirkungen der Zeitverschiebung mildern. Am Tag des Wettkampfs kann der Sportler das Gepäckstück mithilfe der Steuereinrichtung so einstellen, dass die aktivierte Lichtsequenz seine Leistungsfähigkeit im Wettkampf stärkt. Nach dem Wettkampf kann der Sportler das Gepäckstück mithilfe der Steuereinrichtung so einstellen, dass eine körperliche Entspannung schneller eintritt und die Erholungsphase optimiert wird.

Ein weiterer Vorteil der vorliegenden Erfindung ist, dass in dem Gehäuse eine Stromversorgungseinrichtung zum Betrieb der Beleuchtungseinrichtung angeordnet ist. Durch die Anordnung einer eigenen Stromversorgungseinrichtung in dem Gehäuse, wird die Voraussetzung geschaffen, das Gepäckstück an nahezu beliebigen Orten zu verwenden und die Stromversorgung dabei sicherzustellen.

Ein weiterer Vorteil der vorliegenden Erfindung ist, dass die Stromversorgungseinrichtung einen wieder aufladbaren Akkumulator aufweist, der in dem Gehäuse integriert ist. Durch die Integration eines wieder aufladbaren Akkumulators in dem Gehäuse kann das Gepäckstück über längere Zeiträume unabhängig vom öffentlichen Stromnetz betrieben werden.

Ein weiterer Vorteil der Folge Erfindung ist, dass die Stromversorgungseinrichtung einen Netzanschluss aufweist. Mit einem solchen Netzanschluss ist es möglich, das Gepäckstück alternativ oder zusätzlich zu dem Akkumulator auch mit Strom aus einem öffentlichen Stromnetz zu versorgen. Zudem kann der wieder aufladbaren Akkumulator über diesen Netzanschluss wieder aufgeladen werden.

Weitere Vorteile der Folge Erfindung ergeben sich aus den Merkmalen der weiteren Unteransprüche.

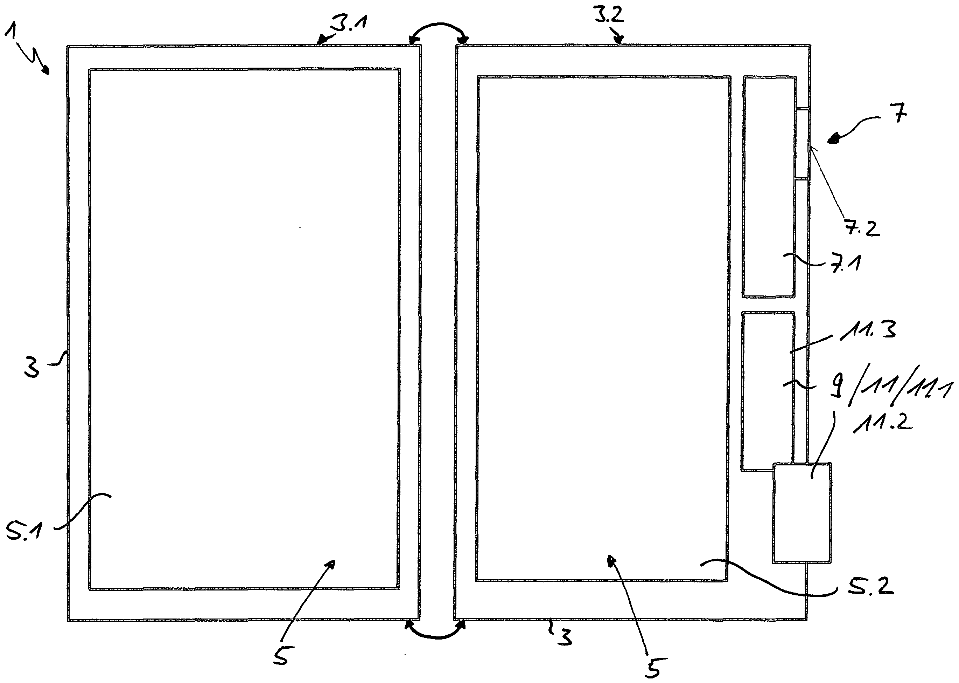

Eine Ausführungsform der vorliegenden Erfindung wird im Folgenden anhand der Zeichnung näher beschrieben. Die einzige Figur der Zeichnung ist eine schematische Draufsicht eines aufgeklappt Gepäckstücks gemäß vorliegender Erfindung.

In der Figur ist ein Gepäckstück 1 gemäß vorliegender Erfindung mit einem Gehäuse 3 in einem aufgeklappt Zustand schematisch dargestellt. In der dargestellten Ausführungsform handelt es sich bei dem Gepäckstück 1 um einen Hartschalenkoffer, zum Beispiel aus Aluminium, mit einem ersten Gehäuseteil 3.1 und einem zweiten Gehäuseteil 3.2. In anderen Ausführungsformen kann das Gepäckstück 1 auch aus einem anderen Material hergestellt sein, mehr als zwei Gehäuseteile aufweisen oder eine andere Geometrie haben. Die allgemeine Ausgestaltung des Gepäckstücks 1 ist nicht Gegenstand der vorliegenden Erfindung.

In dem Gepäckstück 1 ist eine Beleuchtungseinrichtung 5 integriert. In der vorliegenden Ausführungsform umfasst die Beleuchtungseinrichtung 5 eine erste Leuchte 5.1 und eine zweite Leuchte 5.2. Die erste Leuchte 5.1 ist in dem ersten Gehäuseteil 3.1 angeordnet und die zweite Leuchte 5.2 ist in dem zweiten Gehäuseteil 3.2 angeordnet, die in der vorliegenden Ausführungsform jeweils im Wesentlichen eine Gehäusehälfte bilden. In anderen Ausführungsformen kann die Beleuchtungseinrichtung 5 auch nur eine einzige Leuchte 5.1 (Leuchte 5.1 oder Leuchte 5.2) oder mehr als zwei Leuchten umfassen. Die Anzahl der Leuchten ist für die vorliegende Erfindung nicht maßgebend. Eine Beleuchtungseinrichtung 5 mit mehreren Leuchten kann allerdings dazu beitragen, dass die Leuchtwirkung für den Benutzer optimiert wird, weil z.B. ein Raum besser ausgeleuchtet werden kann. Die Beleuchtungseinrichtung 5 weist eine 2-oder mehrkanalige Lichtquelle auf, wobei sie wenigstens einen Melanopsin aktivierenden Lichtkanal (z.B. blau) und wenigstens einen Melanopsin nicht aktivierenden Lichtkanal (z.B. orange) umfasst.

In der zweiten Gehäusehälfte 3.2 ist neben der Leuchte 5.2 eine Stromversorgungseinrichtung 7 angeordnet. Die Stromversorgungseinrichtung 7 umfasst in der vorliegenden Ausführungsform einen wieder aufladbaren Akkumulator 7.1 und einen Netzanschluss 7.2. In anderen Ausführungsformen ist es möglich, nicht-aufladbare Energiespeicher und/oder auch keinen Netzanschluss vorzusehen. Es ist nur besonders vorteilhaft, das Gepäckstück 1 gemäß vorliegender Erfindung unabhängig vom öffentlichen Stromnetz betreiben zu können und den Akkumulator bei Gelegenheit mithilfe des öffentlichen Stromnetzes wieder aufladen zu können.

In der zweiten Gehäusehälfte 3.2 ist zudem eine Steuereinrichtung 9 angeordnet. Die Steuereinrichtung 9 steuert die Beleuchtungseinrichtung 5 und wird ebenfalls von der Stromversorgungseinrichtung 7 mit Strom versorgt. In der dargestellten Ausführungsform sind die Beleuchtungseinrichtung 5, die Stromversorgungseinrichtung 7 und die Steuereinrichtung 9 modulartig in das Gepäckstück 1 bzw. in das Gehäuse 3 integriert.

Die Steuereinrichtung 9 ist mit einer Bedienungseinrichtung 11 verbunden. Die Bedienungsanleitung 11 weist neben einem Ein/Aus-Schalter auch einen Dimmerschalter und optional eine Anzeigeeinrichtung (Display) auf. Die Steuereinrichtung 9 ist in einen programmgesteuerten Rechner (nicht dargestellt) integriert bzw. ein Teil desselben. Aus diesem Grunde weist die Bedienungseinrichtung 11 auch einen Programmwahlschalter auf, über den vorprogrammierte Steuerungen eingestellt werden können. Solche vorprogrammierten Steuerungen können zum Beispiel zeitzonenabhängig, in Abhängigkeit von biologischen Rhythmen oder in einer anderen sinnvollen Abhängigkeit programmiert sein, so dass der Benutzer über die Wahl des Programms eine bestimmte Sequenz, d.h., ein bestimmtes Lichtspektrum und eine bestimmte Lichtintensität, einstellen kann, um sein "System an inneren Uhr" zu stärken. Vorzugsweise umfasst die Beleuchtungseinrichtung 5 eine erste Lichtsequenz zur Stärkung circadianer Rhythmen, eine zweite Lichtsequenz zur akuten Stärkung der Leistungsfähigkeit und eine dritte Lichtsequenz zur Unterstützung der Entspannung des menschlichen Körpers. Die vorliegende Erfindung ist nicht auf diese drei Lichtsequenzen beschränkt, vielmehr können Wahl frei je nach Bedarf beliebiges Lichtsequenzen über das Bedienen fällt manuell eingestellt oder bereits bei der Fertigung vorprogrammiert werden.

### Bezugszeichenliste

- 1: Gepäckstück
- 3: Gehäuse
- 3.1: erster Gehäuseteil
- 3.2: zweiter Gehäuseteil
- 5: Beleuchtungseinrichtung
- 5.1: erste Leuchte
- 5.2: zweite Leuchte
- 7: Stromversorgungseinrichtung
- 7.1: Akkumulator
- 7.2: Netzanschluss
- 9: Steuereinrichtung
- 11: Bedienungseinrichtung
- 11.1: Dimmerschalter
- 11.2: Programmwahlschalter
- 11.3: Anzeigeeinrichtung

## Patentansprüche

1. Gepäckstück mit wenigstens einem Gehäuse (3), in welches eine Beleuchtungseinrichtung (5) integriert ist, mit einem ersten Gehäuseteil (3.1) und einem zweiten Gehäuseteil (3.2), die wenigstens teilweise lösbar miteinander verbunden sind, und mit einer Steuereinrichtung (9) zur Steuerung der Beleuchtungseinrichtung (5),
**dadurch gekennzeichnet,**
**dass** die Steuereinrichtung (9) die Beleuchtungseinrichtung (5) derart steuert, dass diese den circadianen Rhythmus des Benutzers verschiebt, wobei die Beleuchtungseinrichtung (5) eine mehrkanalige Lichtquelle mit wenigstens einem Melanopsin aktivierenden Lichtkanal und wenigstens einem Melanopsin nicht aktivierenden Lichtkanal umfasst.

2. Gepäckstück nach Anspruch 1,
**dadurch gekennzeichnet,**
**dass** in dem Gehäuse (3) eine Stromversorgungseinrichtung (7) zum Betrieb der Beleuchtungseinrichtung (5) angeordnet ist.

3. Gepäckstück nach Anspruch 2,
**dadurch gekennzeichnet,**
**dass** die Stromversorgungseinrichtung (7) einen wieder aufladbaren Akkumulator (7.1) aufweist, der in dem Gehäuse (3) integriert ist.

4. Gepäckstück nach Anspruch 2 oder 3,
**dadurch gekennzeichnet,**
**dass** die Stromversorgungseinrichtung (7) einen Netzanschluss (7.2) aufweist.

5. Gepäckstück nach einem der vorstehenden Ansprüche,
**dadurch gekennzeichnet,**
**dass** die Beleuchtungseinrichtung (5) eine erste Leuchte (5.1) und eine zweite Leuchte (5.2) umfasst, wobei die erste Leuchte (5.1) in dem ersten Gehäuseteil (3.1) und die zweite Leuchte (5.2) in dem zweiten Gehäuseteil (3.1) angeordnet ist.

6. Gepäckstück nach Anspruch 5,
**dadurch gekennzeichnet,**
**dass** die erste Leuchte (5.1) und die zweite Leuchte (5.2) jeweils einen Flächenstrahler bilden.

7. Gepäckstück nach einem der vorstehenden Ansprüche,
**dadurch gekennzeichnet,**
**dass** die Steuereinrichtung (9) eine Dimmerschaltung für die Beleuchtungseinrichtung (5) umfasst.

8. Gepäckstück nach einem der vorstehenden Ansprüche,
**dadurch gekennzeichnet,**
**dass** die Steuereinrichtung (9) in einen programmgesteuerten Rechner integriert ist.

9. Gepäckstück nach einem der Ansprüche 6 bis 8, sofern abhängig von Anspruch 5,
**dadurch gekennzeichnet,**
**dass** die erste und zweite Leuchte (5.1; 5.2) aus dem jeweiligen ersten bzw. zweiten Gehäuseteil (3.1; 3.2) ausklappbar ist.

10. Gepäckstück nach einem der vorstehenden Ansprüche,
**dadurch gekennzeichnet,**
**dass** das Gehäuse (3) aus einem wärmeableitenden Material hergestellt ist.

11. Gepäckstück nach einem der vorstehenden Ansprüche,
**dadurch gekennzeichnet,**
**dass** das Gehäuse (3) eine feste Schale aufweist.

12. Gepäckstück nach Anspruch 11,
**dadurch gekennzeichnet,**
**dass** das Gehäuse (3) im Wesentlichen aus Aluminium hergestellt ist.

## Claims

1. A piece of luggage comprising at least one housing (3) in which an illuminating device (5) is integrated, comprising a first housing part (3.1) and a second housing part (3.2) which are at least partially detachably connected to each other, and comprising a control device (9) for controlling the illuminating device (5),
**characterized in that**
the control device (9) controls the illuminating device (5) such that said illuminating device shifts the circadian rhythm of the user, wherein the illuminating device (5) comprises a multi-channel light source with at least one melanopsin-activating light channel and at least one non-melanopsin-activating light channel.

2. The piece of luggage according to Claim 1, **characterized in that** a power supply device (7) for operating the illuminating device (5) is arranged in the housing (3).

3. The piece of luggage according to Claim 2, **characterized in that** the power supply device (7) has a rechargeable battery (7.1) which is integrated in the housing (3).

4. The piece of luggage according to Claim 2 or 3, **characterized in that** the power supply device (7) has a mains connection (7.2).

5. The piece of luggage according to one of the preceding claims, **characterized in that** the illuminating device (5) comprises a first lamp (5.1) and a second lamp (5.2), wherein the first lamp (5.1) is arranged in the first housing part (3.1) and the second lamp (5.2) is arranged in the second housing part (3.1).

6. The piece of luggage according to Claim 5, **characterized in that** in each case the first lamp (5.1) and the second lamp (5.2) form a planar emitter.

7. The piece of luggage according to one of the preceding claims, **characterized in that** the control device (9) comprises a dimmer circuit for the illuminating device (5) .

8. The piece of luggage according to one of the preceding claims, **characterized in that** the control device (9) is integrated in a programme-controlled computer.

9. The piece of luggage according to one of Claims 6 to 8, when dependent on Claim 5, **characterized in that** the first and second lamp (5.1; 5.2) may be folded out from the respective first and/or second housing part (3.1; 3.2).

10. The piece of luggage according to one of the preceding claims, **characterized in that** the housing (3) is produced from a heat-dissipating material.

11. The piece of luggage according to one of the preceding claims, **characterized in that** the housing (3) has a fixed shell.

12. The piece of luggage according to Claim 11, **characterized in that** the housing (3) is substantially produced from aluminium.

## Revendications

1. Bagage comportant au moins un logement (3) dans lequel est intégré un dispositif d'éclairage (5), avec une première partie de logement (3.1) et une deuxième partie de logement (3.2) qui sont au moins partiellement reliées l'une à l'autre de manière amovible, et avec un dispositif de commande (9) pour la commande du dispositif d'éclairage (5), **caractérisé en ce que** le dispositif de commande (9) commande le dispositif d'éclairage (5) de manière à décaler le rythme circadien de l'utilisateur, dans lequel le dispositif d'éclairage (5) comprend une source lumineuse multicanaux avec au moins un canal lumineux activant la mélanopsine et au moins un canal lumineux n'activant pas la mélanopsine.

2. Bagage selon la revendication 1, **caractérisé en ce qu'**un dispositif d'alimentation (7) pour faire fonctionner le dispositif d'éclairage (5) est disposé dans le logement (3).

3. Bagage selon la revendication 2, **caractérisé en ce que** le dispositif d'alimentation (7) comporte un accumulateur rechargeable (7.1) qui est intégré dans le logement (3).

4. Bagage selon la revendication 2 ou 3, **caractérisé en ce que** le dispositif d'alimentation électrique (7) comporte une alimentation électrique (7.2).

5. Bagage selon une des revendications précédentes, **caractérisé en ce que** le dispositif d'éclairage (5) comprend une première lampe (5.1) et une deuxième lampe (5.2), dans lequel la première lampe (5.1) est disposée dans la première partie de logement (3.1) et la deuxième lampe (5.2) est disposée dans la deuxième partie de logement (3.1).

6. Bagage selon la revendication 5, **caractérisé en ce que** la première lampe (5.1) et la deuxième lampe (5.2) forment chacune un émetteur surfacique.

7. Bagage selon une des revendications précédentes, **caractérisé en ce que** le dispositif de commande (9) comprend un circuit variateur pour le dispositif d'éclairage (5).

8. Bagage selon une des revendications précédentes, **caractérisé en ce que** le dispositif de commande (9) est intégré dans un calculateur commandé par programme.

9. Bagage selon une des revendications 6 à 8, en dépendance de la revendication 5, **caractérisé en ce que** la première et la deuxième lampe (5.1 ; 5.2) peuvent être dépliées de la première ou de la deuxième partie de logement (3.1 ; 3.2) respective.

10. Bagage selon une des revendications précédentes, **caractérisé en ce que** le logement (3) est fabriqué dans un matériau dissipateur de chaleur.

11. Bagage selon une des revendications précédentes, **caractérisé en ce que** le logement (3) présente une coque fixe.

12. Article de bagage selon la revendication 11, **caractérisé en ce que** le logement (3) est fabriqué essentiellement en aluminium.
